(19) Europäisches Patentamt / European Patent Office / Office européen des brevets

(11) **EP 3 856 897 B1**

(12) **EUROPEAN PATENT SPECIFICATION**

(45) Date of publication and mention
of the grant of the patent:
**22.01.2025 Bulletin 2025/04**

(21) Application number: **19835559.6**

(22) Date of filing: **08.10.2019**

(51) International Patent Classification (IPC):
**C12N 9/10** *(2006.01)* **C12Q 1/48** *(2006.01)*

(52) Cooperative Patent Classification (CPC):
**C12N 9/1088; C07K 1/22; C12Q 1/48;
C12Y 205/01018;** C07K 2319/21

(86) International application number:
**PCT/TR2019/050839**

(87) International publication number:
**WO 2020/096550 (14.05.2020 Gazette 2020/20)**

(54) **RECOMBINANT TETRAHYMENA THERMOPHILA GLUTATHIONE S TRANSFERASE MU34 ENZYME (TTGSTM34-8XHIS) AND USES THEREOF**

REKOMBINANTES GLUTATHION-S-TRANSFERASE-MU34-ENZYM (TTGSTM34-8XHIS) AUS TETRAHYMENA THERMOPHILA UND DESSEN VERWENDUNGEN

ENZYME GLUTATHION-S-TRANSFÉRASE MU34 RECOMBINANTE DE TETRAHYMENA THERMOPHILA (TTGSTM34-8XHIS) ET UTILISATIONS ASSOCIÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.11.2018 TR 201816554**

(43) Date of publication of application:
**04.08.2021 Bulletin 2021/31**

(73) Proprietor: **Anadolu Universitesi
Tepebasi/Eskisehir (TR)**

(72) Inventors:
• **ARSLANYOLU, Muhittin**
  **Eskisehir (TR)**
• **KAPKAC, Handan Acelya**
  **Tepebasi/Eskisehir (TR)**

(74) Representative: **Sevinç, Cenk
Grup Ofis Marka Patent A.S.
Atatürk Bulvari, 211/11
Kavaklidere
06680 Ankara (TR)**

(56) References cited:
• **DATABASE UniProt [online] 3 October 2012
(2012-10-03), "Glutathione S-transferase, amine-
terminal domain protein", XP002798296,
retrieved from EBI accession no. I7M2D2_TETTS
Database accession no. I7M2D2**
• **ARSLANYOLU M ET AL: "Recombinant protein
expression of Tetrahymena thermophila
glutathione-S-transferase zeta (6xHis-GSTz) in E.
coli suggest a possible use as a GSTz or 6xHis-
GSTz dual tag in Tetrahymena protein
expression vectors", NEW BIOTECHNOLOGY,
ELSEVIER BV, NL, vol. 25, 1 September 2009
(2009-09-01), pages S66 - S67, XP026461122,
ISSN: 1871-6784, [retrieved on 20090812], DOI:
10.1016/J.NBT.2009.06.302**

Remarks:
The complete document including Reference Table(s) and the Sequence Listing(s) can be downloaded from the EPO website

EP 3 856 897 B1

## Description

## Technical Field

The present invention relates to the recombinant GST enzyme that is produced in the organism *Tetrahymena thermophila.* More specifically, the present invention relates to TtGSTm34-8xHis enzyme and uses thereof.

## Background of the Invention

*Tetrahymena thermophila* is a ciliated eukaryotic unicellular organism and it is one of the best-characterized unicellular eukaryotes. *Tetrahymena thermophila* bears two nuclei: a transcriptionally inactive, diploid germline nucleus (MIC) and a transcriptionally active, polypiloid somatic macronucleus (MAC). Use of *Tetrahymena thermophila* as a model organism in the fields of molecular, cell and developmental biology has been known for many years. The organism has been on the focus based on its advantages in the field. Among these advantages, short cell division time (under optimal conditions, the generation time is about 1.5-3 hours), fermentation in standard bacterial/yeast equipment, easy/cost-effective laboratory growth, and the ability to introduce post-translational modifications to the protein of interest can be counted. Milestone discoveries made in Tetrahymena include the basic analysis of dynein motors, discovery of catalytic ribonucleic acids (ribozymes), the finding of structure of telomeres and telomerases, and the discovery of function of histone acetyltransferases in transcription.

Glutathione S-transferases (GSTs) are an abundant family of dimeric proteins. They represent a group of detoxification enzymes. GSTs catalyze the reaction of substrate glutathione with electrophiles of both endogenous and xenobiotic origins. The main biological roles of GSTs can be summed up as detoxification and protection against oxidative stress. By conjugating glutathione with toxic electrophilic substrates, GSTs give rise to less reactive and more soluble products, which can be excreted from the cells or organisms more easily. This way, GSTs can eliminate the negative effects of various xenobiotics or metabolic by-products that otherwise could be harmful to the cells and damage DNA. Glutathione and CDNB (1-Chloro-2,4-dinitrobenzene) are among the substrates of the GST enzyme. GST isoenzymes are widely distributed in nature, being found in organisms as diverse as bacteria, insects, plants, fish, birds, and mammals. As for *Tetrahymena thermophila,* sequenced macronuclear genome revealed a number of GST genes. Since *Tetrahymena thermophila* is a eukaryotic organism, the GST enzymes coded by these genes undergo post-translational modifications in order to become fully functional. US 5866792 A document relates to the chemically-inducible 27 kD subunit of the enzyme glutathione-S-transferase isoform II (GST-II-27) and sequences encoding it. Said patent application aims to provide transgenic plants with herbicide resistance.

Enzyme kinetics is the study of the chemical reactions that are catalysed by enzymes. Measuring the reaction rate and investigating the effects of various conditions on the enzymatic reaction are in the field of interest of enzyme kinetics. The results obtained by this way can reveal the catalytic mechanism of an enzyme, it's role in the metabolism, and cofactors, inducers and inhibitors of the enzyme.

In a mathematical description of enzyme action, two constants play an important role: $V_{max}$ and $K_m$. $V_{max}$ is the maximum velocity or rate at which an enzyme catalyzes a reaction. The rate of an enzyme-catalyzed reaction does not exceed beyond a certain point even though substrate concentration is increased. This point is reached when there are enough substrate to completely saturate all of the enzyme active sites. $V_{max}$ is the rate of the reaction under these conditions. Higher the $V_{max}$, faster the reaction takes place. The other constant $K_m$ can be defined as the substrate concentration at which half of the enzyme's active sites are filled by substrate. A high $K_m$ value means that the enzyme has low affinity for the substrate, since a high amount of substrate must be present to saturate the enzyme. On the other hand, a low $K_m$ value means a small amount of substrate is necessary to saturate the enzyme, pointing towards a high affinity for substrate. In the context of a paper in which the enzyme kinetics properties of GST enzyme are determined, said GST enzyme RpGSTμ is isolated from the organism *Ruditapes philippinarum.* $K_m$ values of the enzyme for substrates glutathione and CDNB are calculated as 1.03 mM and 0.56 mM, respectively (Bathige et al., "A mu class glutathione S-transferase from Manila clam Ruditapes philippinarum (RpGSTμ): Cloning, mRNA expression, and conjugation assays", 2014*).

Enzyme kinetics test kits enable students and researchers to understand the practical implication of enzyme activity and factors affecting its rate of reaction. Users can be informed about the most important factors which affect the enzyme activity such as enzyme concentration, substrate concentration, pH and temperature of reaction by testing and calculating the conversion rate of the substrate. Measuring the activity of an enzyme over time and under different environmental parameters (temperature, pH, inhibitors, etc) give the users of the kit a practical insight on the kinetics of enzymes. KR 20080041961 A relates to a test kit, which comprises urease enzyme. In the context of this patent application, urease enzyme which is extracted from soybean is characterized including enzyme kinetics properties.

Affinity chromatography (also named as affinity purification) is a separation method based on binding of a molecule in a biochemical mixture to another molecule or ion to which it shows a specific affinity and interaction. The method is based on binding and separation of molecules in solution (mobile phase) from a stationary material (solid phase) due to differences in chemical or physical interaction. A particular ligand is immobilized to a solid support, thus when a mixture of molecules is passed over the column, those molecules which have specific affinity to the ligand bind to the column. This process is followed by washing away the unbound sample components, leaving the molecule of interest bound to the column. Said bound molecule is then purified from the column by the utilization of a solvent that disrupts the ligand interaction.

There are certain affinity tags that are widely used in protein purification. GST is a commonly used affinity tag to aid in the purification of recombinant proteins. GST moiety, which is added at the any end of the protein of interest which is desired to be produced, binds to glutathione fixed to the column with high affinity. This binding is dearranged by the addition of reduced glutathione to the elution buffer, therefore the elution of the GST-tagged target protein can occur. This means that the protein is purified under non-denaturing conditions, which is a necessity in order to obtain a functional protein. A specific protease site can be located between the GST moiety and the protein of interest, by this way separation of GST moiety from the protein can occur effectively in the presence of a certain protease. The more affinity GST has for glutathione means more effectively purification can be achieved.

His-tag (Polyhistidine-tag) is another widely used affinity tag. It can be defined as an amino acid motif in proteins that consists of at least six histidine (His) amino acids. Polyhistidine-tagged recombinant proteins can be purified from a mixture based on His-tag's affinity for certain metal ions such as $Ni^{2+}$, $Co^{2+}$, or $Zn^{2+}$. Among these ions, Ni is the most preferable since His-tag shows high affinity towards Ni. Ni-NTA (nitrilotriacetic acid) beads can be immobilized on a column in order to obtain relatively pure protein (NTA is acting as a chelating group). Bound proteins can be released from the matrix using imidazole.

In consideration of the points mentioned above, a GST enzyme that has high affinity for its substrates, and has mild optimum enzyme activity conditions would be an improvement in the fields of enzyme kinetics and affinity purification.

## Summary of the Invention

The present invention relates to a recombinant *Tetrahymena thermophila* Glutathione S Transferase Mu34 (TtGSTm34-8xHis) enzyme encoded by nucleic acid sequence comprising 8xHis tag which is at least 95% homologous to SEQ ID NO: 1. The TtGSTm34-8xHis enzyme according to the current invention comprises the GST enzyme base sequence encoded by the nucleic acid sequence as given in SEQ ID NO: 3. In preferred embodiments of the invention, said recombinant TtGSTm34-8xHis enzyme encoded by a nucleotide sequence which is identical to SEQ ID NO: 1.

In another aspect, the present invention relates to a test kit for practising enzyme kinetics comprising the TtGSTm34-8xHis enzyme.

In a further aspect, the present invention relates to a vector comprising the TtGSTm34-8xHis enzyme mentioned above. Said vector preferably comprises pIGF-1 base vector.

In another aspect, the present invention provides the use of the recombinant TtGSTm34-8xHis enzyme disclosed herein for determining the enzyme kinetics or for enzyme kinetics practise.

Still in another aspect, the present invention provides the use of the recombinant TtGSTm34-8xHis enzyme disclosed herein as an affinity tag for the purification of proteins. Said use can be dual affinity purification of proteins.

Still in another aspect, the present invention provides the use of fusion proteins that are tagged with TtGSTm34-8xHis by the injection of the protein as an antigen to the animals like mouse or rabbit, in the production of monoclonal and polyclonal primary antibody that are needed in the Western Blotting analyses.

The present invention also provides a method for purifying proteins with the use of recombinant TtGSTm34-8xHis enzyme as an affinity tag, comprising the steps of:

- tagging the C terminal of the protein to be purified with the recombinant TtGSTm34-8xHis enzyme by the usage of PmeI and XhoI restriction sites of pIGF-1-GSTMu34 vector,
- performing affinity chromatography using His-tag, and
- using GST tag affinity chromatography for further purification.

In a detailed explanation of the invention, the recombinant enzyme of the invention is produced by using the pIGF-I vector. The vector is constructed to comprise TtGSTm34 gene sequence, enterokinase site and 8xHis-tag sequence. Said vector is then transformed into host *Tetrahymena thermophila* cells by electroporation, in order for the recombinant enzyme to be expressed.

## Brief Description of the Figures

Figure 1 shows the original pIGF-I-GFP vector and the pIGF-1-GSTMu34 vector that is constructed upon the original vector. A) Original pIGF-I-GFP vector and the general structure. B) pIGF-1-GSTMu34 vector that is constructed by the deletion of the GFP coding sequence of the pIGF-I-GFP vector and insertion of GST and 8xHis coding sequences, and the general structure. C) The agarose gel image of the DNA fragment that contains TtGSTm34-8xHis that is released by the restriction of pIGF-1-GSTMu34 vector with PmeI and ApaI restriction enzymes.

Figure 2 shows the time-dependent induction of a positive transformant cell with $CdCl_2$ and SDS-Page gel analysis of total protein isolates that were purified in a time-dependent manner.

Figure 3 shows the SDS-Page gel analysis of GSTMu34-8xHis that was purified from the total protein isolate using Ni-NTA beads. The transformant cells were incubated for 30 hours with $CdCl_2$. (TP: Total Protein, FT: Unbound proteins, W1 and W3: Proteins that were released after washings, E1-E3: First, second and third elutions that contain purified GSTMu34-8xHis, NegE: Ni-NTA elution of pIGF-TtGSTm34 bearing transformant cells that were not induced with $CdCl_2$, PozGST: Commercial positive control GST enzyme (Cayman Glutathione S-Transferase Assay Kit, Item No: 703302)).

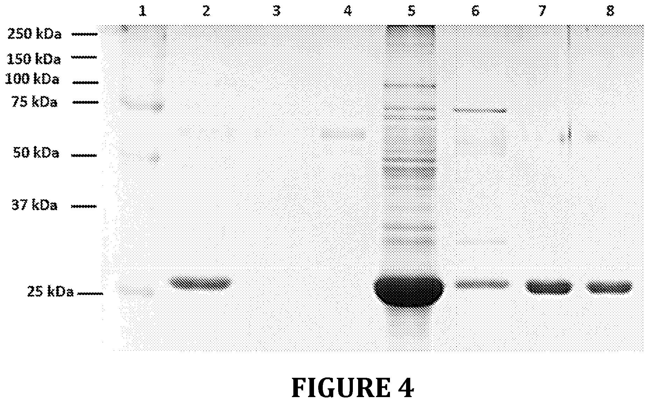

Figure 4 shows the SDS-Page gel analysis of recombinant GSTMu34-8xHis that was purified by dual affinity purification and ultrafiltration from total protein derived from pIGF-TtGSTm34 bearing transformant cells that were subjected to $CdCl_2$ induction. (1: Marker (Biorad Precision Plus Protein™ Unstained Protein Standards Cat No:1610363), 2: First elution after the purification with Ni-NTA beads, 3 and 4: Soluble proteins that passed through filter after the first and second centrifuge during the ultrafiltration, 5: Proteins that were collected at the ultrafiltration filter, 6: Proteins that were not bound to GSH affinity beads, 7 and 8: First and second elutions of proteins that were purified using the GSH affinity beads).

Figure 5 shows the effect of pH on TtGSTm34-8xHis enzyme activity graphically.

Figure 6 shows the effect of ionic strength on TtGSTm34-8xHis enzyme activity graphically.

Figure 7 shows the effect of temperature on TtGSTm34-8xHis enzyme activity graphically.

Figure 8 shows the Lineweaver burk graph of recombinant TtGSTm34-8xHis enzyme for substrate CDNB.

Figure 9 shows the Lineweaver burk graph of recombinant TtGSTm34-8xHis enzyme for substrate GSH.

## Detailed Description of the Invention

In this detailed description, the GST enzyme of the invention (TtGSTm34-8xHis) and uses thereof for specific purposes are described only for better understanding of the subject matter.

The GST enzyme of the present invention can be useful for the enzyme kinetics kits. This enzyme can also be used in affinity purification of recombinant proteins. Said enzyme can also be used for dual affinity purification of proteins. The enzyme may further be used as a positive control in SDS-PAGE and Western Blot analyses.

Said GST enzyme sequence was derived from unicellular eukaryotic organism *Tetrahymena thermophila* and was produced in this organism as well as recombinant TtGSTm34-8xHis. The aim is to obtain a recombinant GST enzyme that shows high affinity towards its substrates. Moreover, said GST enzyme's effectivity and stability should be high in order to provide an advantage over commercially available GST enzymes.

Recombinant enzymes are produced with the help of vectors. Vector is added with protein sequence of interest in order to be further modified with insertions of tags, signals and restriction sites. The constructed vector is then transferred to the host organism for production of protein of interest. In the context of the present invention, vector of choice is preferably pIGF-I. The original vector contains a sequence coding for GFP (*Y* *Ilmaz G. ve Arslanyolu M., 2015. "Efficient expression of codon-adapted affinity tagged super folder green fluorescent protein for synchronous protein localization and affinity purification studies in Tetrahymena thermophila", BMC Biotechnology,* 15:22). The said sequence was released and TtGSTm34 sequence was inserted to the vector instead. Along with the TtGSTm34, 8xHis sequence was added to the vector with an enterokinase site between these two sequences.

*Tetrahymena thermophila* is a well-studied organism in the field of molecular biology. Ability for multiplying to high cell densities with short generation times, being able to be grown in laboratory easily and cost-effectively, ability to modify produced proteins with post-translational modifications are some of the reasons this organism is widely used. Since the recombinant enzyme of the invention (TtGSTm34-8xHis) was both derived from and produced in *Tetrahymena thermophila,* the protein is expected to be functional.

Glutathione S-transferases (GSTs) are a family of detoxification enzymes. They protect the cells/organisms against toxic substances and oxidative stress. They catalyze the reactions involving substrate glutathione and/or xenobiotic substrates. As a result, less reactive products are obtained and the DNA is protected against the negative effects of xenobiotics. In *Tetrahymena thermophila* macronuclear genome, a certain number of GST genes is found. These genes code for the proteins that undergo post-translational modifications in order to become fully functional.

Apart from their natural functions in the cells, GSTs are also used in molecular biology as affinity purification tags. Affinity purification can be defined as use of specific interactions between molecules as a way to resolve mixture of biochemicals. The biochemical mixture is called mobile phase, and the ligand to be bound is found as immobilized (stationary phase). Affinity tags are sequences that are attached to the protein of interest prior to the chromatography. They assure the interaction of recombinant protein with the stationary phase. GSTs are one of the most commonly used tags for this purpose. GST-tagged recombinant protein is separated from the rest of the mixture with the help of GST's affinity to substrate glutathione, which is found affixed in the column.

GST's affinity for glutathione determines the effectivity of the purification. TtGSTm34-8xHis of the invention has a $K_m$ value of 0,54 mM for glutathione. This low $K_m$ value indicates that the TtGSTm34-8xHis has high affinity towards glutathione.

His-tag is also widely used as an affinity purification tag. The efficiency of polyhistidine-tag as an affinity tag is based on His-tag's affinity for certain metal ions. In the context of the invention, $Ni^{2+}$ is used in the form of Ni-NTA beads, and immobilized to the column. Having used affinity tag features of both GST and His-tag, dual affinity purification is performed during the purification of TtGSTm34-8xHis. Ultrafiltration is performed durind dual affinity purification after the first affinity purification in order to change the elution buffer used in the chromatography.

Purified recombinant enzyme is then subjected to enzyme kinetics tests. Enzyme kinetics is the study of enzyme-catalyzed reactions. The rate of reactions between enzymes and substrates, and the parameters (pH, temperature, ionic strength) that affect the reaction are within the scope of enzyme kinetics. $V_{max}$ and $K_m$ values are calculated in order to determine the efficiency of enzyme's catalytic activity. In the context of the present invention, enzyme kinetics tests of TtGSTm34-8xHis for two different substrates (glutathione and CDNB) have been performed.

An enzyme's stability is the extent to which an enzyme retains its activity when subjected to storage, purification and various other physical or chemical manipulations. The stability of TtGSTm34-8xHis is tested for different periods of time, and at various temperatures. The results of specific activity measurements (Table 4) indicate the long-term shelf life of the recombinant enzyme according to the present invention at different temperatures.

In an aspect of the present invention, GST enzyme of *Tetrahymena thermophila* is modified with 8xHis tag, and the resulting recombinant enzyme TtGSTm34-8xHis (which is at least 95% homologous to SEQ ID NO: 1) is produced in the same organism. Recombinant TtGSTm34-8xHis enzyme encoded by a nucleotide sequence which is at least 95% homologous to SEQ ID. NO: 1. In preferred embodiments of the invention, recombinant TtGSTm34-8xHis enzyme encoded by nucleic acid sequence which is identical to SEQ ID NO: 1.

In an aspect of the present invention, TtGSTm34-8xHis enzyme (SEQ ID NO: 1) comprises the nucleotide sequence of GST enzyme as given in SEQ ID NO: 3.

In an aspect of the present invention, there is provided a test kit comprising TtGSTm34-8xHis for the practise of an enzyme kinetics. Said enzyme kinetics test kit can make use of the decrease in the amount of substrate as a way for the measurement of the reaction rate.

In another aspect, the present invention provides the use of TtGSTm34-8xHis as an affinity tag for the purification of proteins.

## A) Cloning of the TtGSTm34 Gene into the Recombinant Protein Production Vector pIGF-1 and Transformation into *Tetrahymena Thermophila* Cells

The protein coding gene sequence (660 bp) and the nucleic acid sequence of TtGSTm34 gene derived from the TGD (Tetrahymena Genome Database) is given in SEQ ID NO: 3 (Gene ID: 7835917). The cDNA ORF of the GSTm34 gene in the pIGF-1-GSTm34 vector was designed as a 722 bp recombinant DNA sequence, which was added restriction enzymes, enterokinase and 8xHis tag. The cloning can be carried out using restrictions with Pmel and Apal. Thus if desired, the 8xHis affinity tag of the purified TtGSTm34 can be removed by restriction with the entrokinase protease enzyme. With this vector design, GFP gene which resides in cadmium-inducible MTT1-GFP-rpl29 protein production gene cassette is removed from the vector using restrictions with Pmel and Apal and instead of GFP gene, 722 bp TtGSTm34-8xHis, which was cut with Pmel and Apal is ligated into the vector. DNA Sequence of cloned recombinant TtGSTm34-8xHis is as follows:

**SEQ ID NO:1**

5′(gtttaaac)(ctcgag)ATGACAACTCTTGGTTACTGGGGCATCAGAGGTTTGGCTCAACCTATCCGTTTCT
TACTTGCCTACTTAGGTGTCTAATACACTAATAAGGCCTACGCCAATCCTGAAGAATGGTTCGGAAAG
GATAAGAATGAACTCGGCTTTGACTTCCCCAACATTCCCTACTTAATTGATGGTGATCTTAAACTTACT
GAATCTTCTGCTATTCCTATTTATCTTATCAGAAAGCACAAAAGAAACGAGTTGTTAGGATCTTCTGC
TGACGGTTCTTACAGTGAAAAGGAAGTTAGAGTCGCTTAAATTGTTGGTGTTATTAGAGATCTTTTTA
AGGAACTCACAGGCTTATGTTTCAACCCTGACTTCAAAAACATTAAAGAAAAGCTCTACACTGAAAAG
CTCGAACTCTTAATTAAGAGACTCGGTGCTTATCTTGGTGATAAGGAATTCATAGTTGGTACCTTGAC
TTATGCTGATTTTCTTTTCTATGAAGCCCTCAGCTATATTAGACATATTTATCCCCAAGCCATTTGTGC
TACTCTTACTGCTTATATCAACAGATTTGAAAACCTCCCTGGTATTAAGGAATACATTGCTAGCCATG

CCTAAGAACTTAAGGTTTTCTTACCTCCTCAAAGAGCTACCTGGTCTGGTCCTTAA(gacgacgacga)(caa

acaccaccaccaccaccaccaccac)tgatga(gggccc)3'

In this sequence, the first parenthetical represents Pmel restriction site, the second parenthetical represents Xhol restriction site, the third parenthetical represents enterokinase restriction site, the fourth parenthetical represents 8xHis tag, the fifth parenthetical represents Apal restriction site.

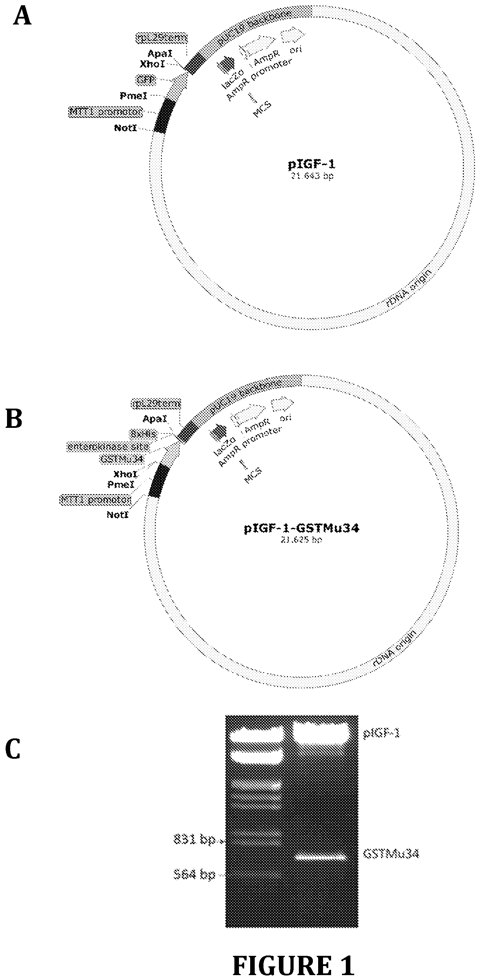

The structures of pIGF-1 expression vector, and pIGF-1-GSTm34 vector that is contstructed upon this vector are given in Figure 1 (Figure 1-A and Figure 1-B).

Total RNA isolation from *Tetrahymena thermophila* was performed with the Qiagen RNeasy Mini Kit (Cat No. / ID: 74104). Total RNA was then treated with DNase (Promega RQ1 RNase-Free DNase Cat No: M6101). In a reaction that was carried out using RevertAid First Strand cDNA synthesis kit (Cat No: K1621, Fermentas), ~2,5 $\mu$g mRNA was used and cDNA is synthesized. Subsequently, PCR was performed with the following gene primers and the target TtGSTm34 protein-encoding region was amplified from the cDNA obtained. The sequence information of the primers used is given below;

FGSTmu34pV2: 5' CGCGTTTAAACCTCGAGATGACAACTCTTGGTTACTGGGGCATCAG 3'

R1GSTmu34pV2: 5' TTTGTCGTCGTCGTCTTAAGGACCAGACCAGGTAGCTCTTTGAGG 3'

R2GSTmu34pV2: 5' GGGCCCTCATCAGTGGTGGTGGTGGTGGTGTTTGTCGTCGTCGTCTTAAGGACC 3'

Enterokinase restriction site and 8xHis tag can be inserted by performing two consecutive PCR reactions with reverse primers R1GSTmu34pV2 and R2GSTmu34pV2. First PCR reaction was carried out using FGSTmu34pV2 and R1GSTmu34pV2 primers and obtained DNA was purified from the gel (Thermo, GeneJet Gel Extraction Kit, #K0691). By using this DNA sequence as a template second PCR reaction in which FGSTmu34pV2 and R2GSTmu34pV2 primers were used is performed. The ends of the resulting 728 bp purified DNA fragment were cut with restriction enzymes Pmel (NEB, # R0560S) and Apal (NEB, # R0114S) in order to acquire sticky ends. pIGF-1 vector was also cut using Pmel and Apal to obtain sticky ends. Cut pIGF-1 vector and 722 bp DNA containing GSTm34-8xHis were visualized on 0.7% agarose gel (Figure 1-C), cut under the UV cabinet of 365 nm wavelength and purified from the gel using the purification kit (Thermo, GeneJet Gel Extraction Kit, # K0691). Sticky end TtGSTm34 gene and pIGF-1 vector were ligated with T4 DNA ligase enzyme (NEB, M0202S), and transformed to E. coli XL1-Blue competent cells using the heat shock. Upon transformation, positive colonies were selected from the Amp plates using Colony PCR method. The plasmids that were isolated from the positive colonies were confirmed using the restrictions with Pmel and Apal.

***Transformation of Tetrahymena Cells by Electroporation Method and Antibiotic Selection***

The vectors transformed to Tetrahymena B2086 - CU428 cells that were freshly evoked from liquid nitrogen with BioRad Gene Pulser device. Tetrahymena cells were transformed and modified by electroporation based on the method of J. Gaertig and M. Gorovsky. Tetrahymena cells that were grown to late log pahse were washed with 10 mM Tris-HCl (pH 7.5). Then the cells were taken to 18-24 hours starvation and the cell numbers were adjusted as $3\times10^5$ per ml. It was then taken up in equal volumes from both cell types, blended into a erlenmeyer flask and taken to conjugation in a static incubator at 30°C to start the conjugation. Conjugation efficiency at the 3rd hour of the conjugation was checked on a light microscope. When the efficiency reached to 80%, the conjugates which were in the "Macronucleus Growth 1" phase were washed with 10 mM HEPES. On the 7th hour of the conjugation, the nuclei of the cells were dyed with Hoechst dye and the cells were observed under the fluorescence microscope. On the 9th hour of the conjugation, 100 ml of cells was centrifuged at 6.500 g for 7 minutes, and the pellet was washed with 10 mM HEPES (pH 7.5). Then the conjugates were pelleted and dissolved with 10 mM HEPES to reach final concentration of $1.7 \times 10^7$ cells/ml. 230 $\mu$l of cells was added 20 $\mu$l 10 mM HEPES which contains 10-40 $\mu$g plasmid, and reached the final volume of 250 $\mu$l. The mixture is then trasferred to Gene Pulser (Bio-Rad) electroporation cuvette with 4 mm width gaps and electrical pulse was applied (440 Volt, 25-$\mu$F Capacitor, 200 $\Omega$). Thereafter, the cells were transferred to SPP medium that contains 5 ml 250 $\mu$g/ml Penicilline-Streptomycin (Pen-Strep). After 12-24 hours static incubation, transformant cells were taken to 60 $\mu$g/ml paromomycin pressure. Amount of paromomycin was increased to 100 $\mu$g/ml on the third day of the conjugation, and to 120 $\mu$g/ml on the fifth day of the conjugation. Positive transformant cells were selected and frozen in tubes of liquid nitrogen that contain DMSO.

### Induction of Transformant Cells with CdCl$_2$

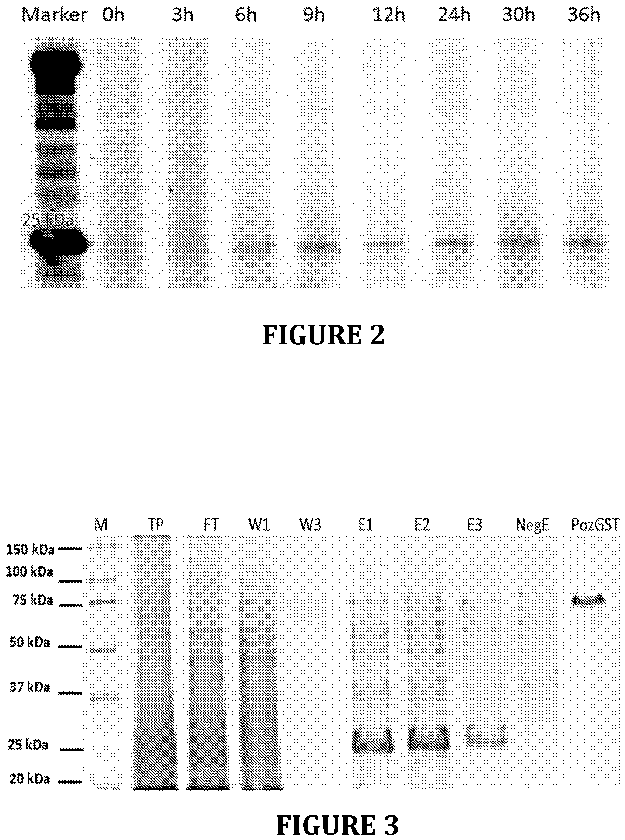

Transformant cells were induced with CdCl$_2$ (Sigma, 439800) to the final concentration of 5$\mu$g/ml for 36 hours. The samples were collected at different intervals and total protein isolation was performed. SDS-PAGE images of the isolates are given in Figure 2. T100B was used as lysis buffer in protein isolation (Final of 1% TritonX100, 2.5 mM Tris-HCl pH 7.5, 150 mM EDTA, 2 mM PMSF, 1X Protease inhibitor Cocktail Cat no: 04693159001 ROCHE).

### Isolation of Protein from Transformant Cells Using Affinity Protein Purification, SDS-PAGE and Western Blot Analyses

30 hours after the cadmium induction of transformant cells, total protein isolation from the cells was carried out. With the use of 8xHis coding sequence that was placed at the 5' end of TtGSTm34 gene, 8xHistidine tag is attached to the C terminal of TtGSTm34 protein and affinity protein purification based on 8xHis tag of TtGSTm34-8xHis was performed using Ni-NTA beads (Cat No:745400.25, Protino Ni-NTA Agarose). The samples that were collected at every phase of purification were loaded onto the SDS-PAGE gel. The beads were washed 3 times with wash buffer containing 20 mM imidazole and eluted 3 times with elution buffer containing 250 mM Imidazole (Figure 3).

In order to determine the TtGSTm34 protein's affinity towards glutathione, total protein that were obtained from transformant cells were purified using GSH Sepharose 4B beads (GE Healthcare Cat No: 52-2303-00 AK). In this procedure, PBS pH 7.3 (140 mM NaCl, 2.7 mM KCl, 10 mM Na$_2$HPO$_4$, 1.8 mM KH$_2$PO$_4$, pH 7.3) was used as binding buffer. Elution buffer on the other hand was selected as 50 mM Tris-HCl, 10 mM reduced glutathione, pH 8.0. Unbound proteins, washed-off proteins, and the final elutions were loaded onto the 12,5% SDS-PAGE gel. Then Western Blotting was applied using anti-histidine antibody. Primary antibody used in Western Blot analysis was "mouse monoclonal anti-6xHis epitope tag" antibody (Cat No: 200-301-382S, Rockland), and the secondary antibody was" GenScript polyclonal goat anti-Mouse IgG" (H&L) [HRP] antibody.

The presence of proteins other than recombinant proteins in the elution wells, made it mandatory to perform a second purification with another tag (Figure 3). For this purpose, the elution tubes that were obtained following purification with Ni-NTA beads were gathered up (total volume of 60 ml). The buffer was replaced using the ultrafiltration method to remove the imidazole present in the elution buffer (Amicon® Ultra-15 10K (cut-off 10kDa) Millipore Cat No: UFC901008). Since the capacity of ultrafiltration tubes are 15 ml, centrifuge was performed four times from the same tube. Thus, the negative effect of imidazole on binding of recombinant protein to GSH beads in the next purification step of purification with GSH beads has been eliminated. After ultrafiltration, a second affinity purification with GSH beads was performed with the collected proteins. The last elution step was performed twice. The resulting proteins that were obtained by purification of the recombinant TtGSTm34-8xHis protein with both Ni-NTA and GSH beads were loaded onto 12,5% SDS-PAGE gel (Figure 4).

B) Enzyme Kinetics of the Recombinant TtGSTm34-8xHis Protein

Determination of GST enzyme activities of affinity purified proteins was performed spectrophotometrically in the presence of 1-chloro-2,4-dinitrobenzene (CDNB) as substrate. 20 $\mu$l of each sample were taken and mixed with 180 $\mu$l of PBS (pH 6.5) buffer (final concentration prepared in 1 mM CDNB Absolute ethanol) and 1 mM reduced Glutathione (prepared in distilled water). The enzyme activities were measured with BioTek The Synergy ™ HT Elisa device using Gen5 software. The O.D values for enzyme activity were determined at 340 nm for 30 minutes with 2 minutes intervals, using BioTek The Synergy™ HT Elisa Reader kinetics program.

Unit value of activity is defined as the amount of enzyme that catalyzes 1 $\mu$mol substrate at 25°C (CDNB extinction coefficient$_{340\ nm}$ = 9.6 mM$^{-1}$ cm$^{-1}$). The enzyme activities were first calculated in terms of U/ml. Then these values were divided by protein concentrations that were determined by Bradford method (Pierce™ Coomassie Plus Bradford Assay Reagent Cat no: 23238). This way specific activities were calculated in terms of U/mg.

Table 1 shows the spesific GST activities and purification yields of the proteins that were obtained by Ni-NTA purification, Ultrafiltration, and GSH affinity purification (these values are calculated under non-optimized reaction conditions). The purification fold of the recombinant enzyme is at a high value of 3.166.

**Table 1**

| | Volume (ml) | Total Protein (mg) | Total Activity (Unit) | Specific Activity ($\mu$molmin$^{-1}$ mg protein $^{-1}$) | Purification Fold | Yield |
|---|---|---|---|---|---|---|
| Soluble Lisate | 40 | 137.37 | 9.2 | 0.06 | 1 | 100 |
| Ni-NTA Purification | 60 | 2.34 | 5.4 | 2.30 | 38.33 | 58.69 |
| Ultrafiltration | 0.25 | 0.08 | 3.42 | 42.75 | 712.5 | 37.17 |
| GSH Affinity Purification | 0.1 | 0.012 | 2.28 | 190 | 3166.66 | 24.78 |

*Enzyme Kinetics Experiments*

Optimum pH, optimum ionic strength and optimum temperature of the recombinant TtGSTm34-8xHis enzyme were determined, and then $K_m$, $V_{max}$, $K_{cat}$ and $K_{cat}/K_m$ values were calculated.

Determination of optimum pH:

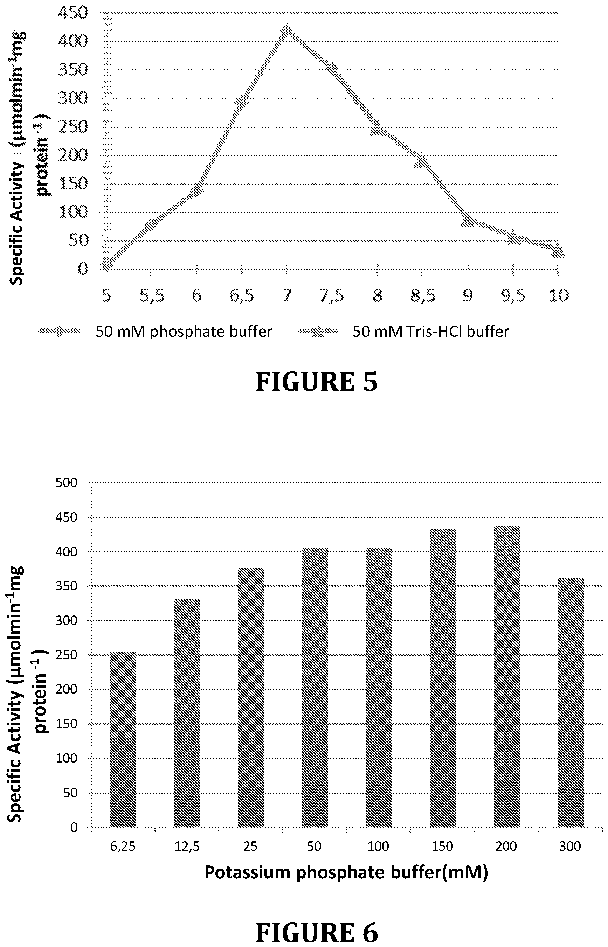

To determine the optimum pH of the recombinant TtGSTm34-8xHis enzyme, 0.1M phosphate buffers with pH 5.0, 5.5, 6.0, 6.5, 7.0, 7.5 and 0.1 M Tris-HCl buffers with pH 8.0, 8.5, 9.0 and 10 were prepared. Measurements of enzyme activity were performed with each buffer, and the results are shown in Figure 5. The optimum pH of the enzyme was found to be 7.0.

Determination of optimum ionic strength:

0.00625 M, 0.0125 M, 0.025 M, 0.05 M, 0.1 M, 0.15 M, 0.2 M and 0.3 M potassium phosphate buffers were prepared to determine the ionic strength at which the recombinant TtGSTm34-8xHis enzyme shows optimum activity. The pH values of these solutions were adjusted to the optimal pH of 7.0, and the activity measurements were made for each solution. The results are shown in Figure 6. The optimum ionic strength of the enzyme was found to be 200 mM.

Determination of optimum temperature:

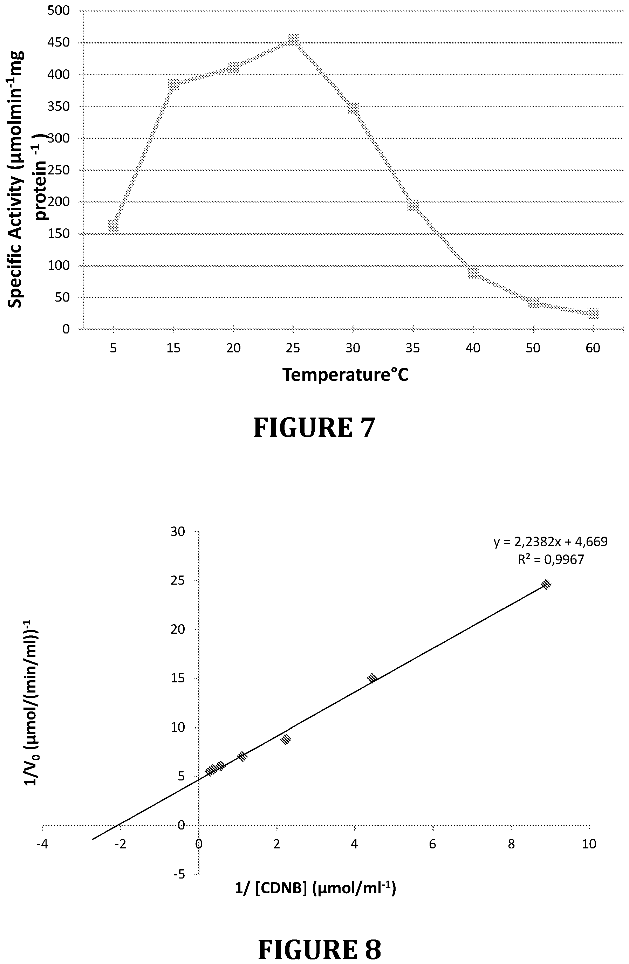

In order to determine the temperature that the recombinant TtGSTMu34-8xHis enzyme shows optimum activity, 0.2M potassium phosphate buffer (pH: 7.0) was used. The buffer contained the cofactors and substrates (final 1 mM CDNB and 1mM GSH) required for activity measurement. The mixture was kept for 5 minutes in a well that was adjusted to a desired temperature. Measurements of activity at temperatures 5°C, 15°C, 20°C, 25°C, 30°C, 35°C, 40°C, 50°C and 60°C were carried out and the results are given in Figure 7. The optimum temperature of the enzyme was found to be 25°C.

Determination of $K_m$, $V_{max}$, $K_{cat}$ and specificity constant ($K_{cat}/K_m$) for the substrates GSH and CDNB:

In the first set of experiments that aim to determine the $K_m$ and $V_{max}$ values of the enzyme glutathione S-transferase for GSH and CDNB substrates, the final concentration of GSH was held at 0,9 mM. Then activity measurements were carried out in 200 mM potassium phosphate buffer at 25°C for 7 different CDNB concentrations: 0,1125 mM, 0,225 mM, 0,45 mM, 0,9 mM, 1,8 mM, 2,7 mM ve 3,6 mM. A $1/V^{-1}$ [CDNB] Lineweaver-Burk graph was drawn according to the results obtained (Figure 8). With this graphical aid, $K_m$ and $V_{max}$ values for CDNB were determined.

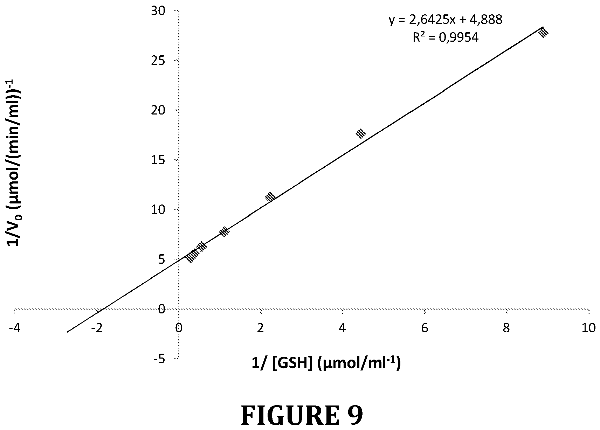

Similarly, 7 different concentrations of GSH (0.1125 mM, 0.225 mM, 0.45 mM, 0.9 mM, 1.8 mM, 2.7 mM and 3.6 mM) in the constant CDNB concentration of 0.9 mM was used. The activity measurements were made under the same conditions and $1/V^{-1}$[GSH] graph was drawn (Figure 9). $K_m$ and $V_{max}$ values for GSH were determined using this graph. These calculations were followed by quantitative protein assay (Bradford) and total enzyme amount (Et) was determined. $K_{cat}$ value, which reflects the enzyme turnover number was calculated using the formula given below:

$$k_{cat}=V_{max}/E_t$$

One way of comparing the catalytic effects of various enzymes or the conversion of different substrates to the product with the same enzyme is to find the specificity constant ($V_0$) for the reactions. The specificity constant is calculated by the following formula:

$$V_0=k_{cat}/k_m$$

Lineweaver-Burk graphs drawn for both CDNB and GSH are given in Figures 8 and 9, respectively. The values of $K_m$, $V_{max}$, $K_{cat}$ and $V_0$ for CDNB and GSH substrates were calculated with the help of these graphs and the results are given in Table 2.

**Table 2**

| Substrate | $K_m$ (mM) | $V_{max}$ (U/ml) | $K_{cat}$ ($s^{-1}$) | $K_{cat}/K_m$ |
|---|---|---|---|---|
| GSH | 0.54 | 0.2 | 186.9 | 345.3 |
| CDNB | 0.47 | 0.21 | 195.4 | 407.7 |

*Specific Activity Comparison with the Known GST Enzymes and Enzyme Stability Tests*

The next step of the experiments focused on the comparison of the specific activity of TtGSTm34-8xHis of present invention with the specific activities of GST enzymes that are commercially available. For this purpose, the enzyme reaction was carried out under optimum conditions that are determined earlier: 200 mM phosphate buffer concentration, pH 7.0, and 25°C temperature. Under these conditions, the activity of TtGSTm34-8xHis reached to 453 U/mg. Table 3 shows this value compared to specific activities of commercially available GST enzymes.

**Table 3**

| GST | Specific Activity ($\mu$molmin$^{-1}$mg protein$^{-1}$) | Origin |
|---|---|---|
| Schistosoma japonicum GST (LSBio, Cat no: LS-G627) | 2.8 - 3.3 | E. coli |
| Schistosoma japonicum GST (MyBioSource, Cat no: MBS203158) | >20 | E. coli |
| Recombinant human Glutathione S Transferase alpha 1 protein (Abcam, Cat no: ab167981) | 5-8 | E. coli |
| Recombinant human GSTM2 protein (Abcam, Cat no: ab168017) | <25 | E. coli |
| Recombinant mouse Glutathione S Transferase mu protein (Abcam, Cat no: ab168035) | <11 | E. coli |
| GSTM1 glutathione S-transferase (Creative Biomart, Catno: GSTM1-2473H) | 68.9 | E. coli |
| Recombinant Human Glutathione S-transferase Mu 5 Protein (Novusbio, Cat no: NBP2-52145) | >90 | E. coli |
| **Recombinant TtGSTm34-8xHis (The present invention)** | **453** | **Tetrahymena thermophila** |

The stability of TtGSTm34-8xHis was also tested within the scope of experiments. Shelf life analyses of the enzyme were performed under various conditions and intervals. When kept in glycerol and phosphate buffer at -20°C, TtGSTm34-8xHis enzyme stocks lost only 39% of the enzyme activity in a year. When TtGSTm34-8xHis enzyme is kept for 10 days at +4°C, the loss of activity is determined to be 33%. At the room temperature, the enzyme loses 82% of its activity in 2 days. However, the specific activity is kept at 78 U/mg. The results of the enzyme stability tests for TtGSTm34-8xHis are given in Table 4.

**Table 4**

| Time | Temperature | Specific Activity | Loss % |
|---|---|---|---|
| 0 days | Room Temperature (25°C) | 453 | - |
| 2 days | Room Temperature (25°C) | 78 | 82 |
| 10 days | Fridge (4°C) | 302 | 33 |
| 6 months | Deep-freeze (-20°C) | 348 | 23 |
| 12 months | Deep-freeze (-20°C) | 275 | 39 |

SEQ ID NO information of the nucleotide and nucleic acid sequences of recombinant TtGSTm34-8xHis enzyme of the invention and original TtGSTm34 enzyme are given in Table 5.

**Table 5**

| SEQ ID NO: 1 | TtGSTm34-8xHis (DNA) |
|---|---|
| SEQ ID NO: 3 | TtGSTm34 (DNA) |

**Claims**

1. A recombinant Tetrahymena thermophila Glutathione S Transferase Mu34 (TtGSTm34] enzyme encoded by the nucleic acid sequence comprising 8xHis tag (TtGSTm34-8xHis] which is at least 95% homologous to SEQ ID NO: 1.

2. The TtGSTm34-8xHis enzyme according to claim 1, comprising the GST enzyme base sequence encoded by the nucleic acid sequence according to SEQ ID NO: 3.

3. The recombinant TtGSTm34-8xHis enzyme according to claim 1 or 2 encoded by the nucleic acid sequence which is identical to SEQ ID NO: 1.

4. A test kit for practising enzyme kinetics comprising a TtGSTm34-8xHis enzyme according to claim 1.

5. A vector comprising a TtGSTm34-8xHis insertion according to claim 1.

6. A vector according to claim 5 further comprising a pIGF-1 vector.

7. Use of the recombinant TtGSTm34-8xHis enzyme according to claim 1 for determining an enzyme kinetics or for enzyme kinetics practise.

8. Use of the recombinant TtGSTm34-8xHis enzyme according to claim 1 as an affinity tag for the purification of proteins.

9. The use according to claim 8 wherein TtGSTm34-8xHis enzyme is used for dual affinity purification of proteins.

10. Use of the recombinant TtGSTm34-8xHis enzyme according to claim 1 as a positive control in SDS-PAGE and Western Blot analyses or as an antigen protein in the production of monoclonal and polyclonal antibodies.

11. A method for purifying proteins with the use of recombinant TtGSTm34-8xHis enzyme according to claim 1 as an affinity tag, comprising the steps of:

    - tagging the protein to be purified with the recombinant TtGSTm34-8xHis enzyme,
    - performing affinity chromatography by using His-tag as an affinity tag, and
    - using GST tag affinity chromatography for further purification.

**Patentansprüche**

1. Rekombinantes Tetrahymena thermophila-Glutathion-S-Transferase-Mu34 (TtGSTm34)-Enzym, für das die Nukleinsäuresequenz mit 8xHis-Tag (TtGSTm34-8xHis) kodiert, die zu mindestens 95% homolog zu SEQ ID NO: 1 ist.

2. TtGSTm34-8xHis-Enzym nach Anspruch 1, umfassend die GST-Enzym-Basensequenz, die durch die Nukleinsäuresequenz gemäß SEQ ID NO: 3 codiert wird.

3. Rekombinantes TtGSTm34-8xHis-Enzym nach Anspruch 1 oder 2, codiert durch die Nukleinsäuresequenz, die mit SEQ ID NO: 1 identisch ist.

4. Testkit zum Üben der Enzymkinetik, umfassend ein TtGSTm34-8xHis-Enzym nach Anspruch 1.

5. Vektor, umfassend eine TtGSTm34-8xHis-Insertion nach Anspruch 1.

6. Vektor nach Anspruch 5, der ferner einen pIGF-1-Vektor umfasst.

7. Verwendung des rekombinanten TtGSTm34-8xHis-Enzyms nach Anspruch 1 zur Bestimmung einer Enzymkinetik oder zur Durchführung von Enzymkinetiken.

8. Verwendung des rekombinanten TtGSTm34-8xHis-Enzyms nach Anspruch 1 als Affinitätstag für die Reinigung von Proteinen.

9. Verwendung nach Anspruch 8, wobei das TtGSTm34-8xHis-Enzym für die Dual-Affinitäts-Reinigung von Proteinen verwendet wird.

10. Verwendung des rekombinanten TtGSTm34-8xHis-Enzyms nach Anspruch 1 als Positivkontrolle in SDS-PAGE- und Western-Blot-Analysen oder als Antigenprotein bei der Herstellung von monoklonalen und polyklonalen Antikörpern.

11. Verfahren zur Reinigung von Proteinen unter Verwendung von rekombinantem TtGSTm34-8xHis-Enzym nach Anspruch 1 als Affinitätsmarkierung, umfassend die folgenden Schritte:

     - Markierung des zu reinigenden Proteins mit dem rekombinanten TtGSTm34-8xHis-Enzym,
     - Durchführung von Affinitätschromatographie unter Verwendung von His-Tag als Affinitätsmarkierung, und
     - Verwendung von GST-Tag-Affinitätschromatographie zur weiteren Reinigung.


**Revendications**

1. Enzyme recombinante Tetrahymena thermophila Glutathion S Transférase Mu34 (TtGSTm34] codée par la séquence d'acide nucléique comprenant l'étiquette 8xHis (TtGSTm34-8xHis] qui est au moins à 95% homologue à SEQ ID NO : 1.

2. Enzyme TtGSTm34-8xHis selon la revendication 1, comprenant la séquence de base de l'enzyme GST codée par la séquence d'acide nucléique selon SEQ ID NO : 3.

3. Enzyme TtGSTm34-8xHis recombinante selon la revendication 1 ou 2 codée par la séquence d'acide nucléique qui est identique à SEQ ID NO: 1.

4. Kit de test pour pratiquer la cinétique enzymatique comprenant une enzyme TtGSTm34-8xHis selon la revendication 1.

5. Vecteur comprenant une insertion TtGSTm34-8xHis selon la revendication 1.

6. Vecteur selon la revendication 5 comprenant en outre un vecteur pIGF-1.

7. Utilisation de l'enzyme recombinante TtGSTm34-8xHis selon la revendication 1 pour la détermination d'une cinétique enzymatique ou pour la pratique de la cinétique enzymatique.

8. Utilisation de l'enzyme recombinante TtGSTm34-8xHis selon la revendication 1 comme marqueur d'affinité pour la purification de protéines.

9. Utilisation selon la revendication 8, dans laquelle l'enzyme TtGSTm34-8xHis est utilisée pour la purification par double affinité de protéines.

10. Utilisation de l'enzyme recombinante TtGSTm34-8xHis selon la revendication 1 comme contrôle positif dans les analyses SDS-PAGE et Western Blot ou comme protéine antigène dans la production d'anticorps monoclonaux et polyclonaux.

11. Procédé de purification de protéines à l'aide de l'enzyme recombinante TtGSTm34-8xHis selon la revendication 1 comme marqueur d'affinité, comprenant les étapes consistant à :

   - marquer la protéine à purifier avec l'enzyme recombinante TtGSTm34-8xHis,
   - effectuer une chromatographie d'affinité en utilisant l'étiquette His comme étiquette d'affinité, et
   - utiliser la chromatographie d'affinité avec étiquette GST pour une purification supplémentaire.

**FIGURE 1**

**FIGURE 2**

**FIGURE 3**

**FIGURE 4**

**FIGURE 5**

**FIGURE 6**

**FIGURE 7**

**FIGURE 8**

**FIGURE 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5866792 A

- KR 20080041961 A

**Non-patent literature cited in the description**

- **BATHIGE et al.** *A mu class glutathione S-transferase from Manila clam Ruditapes philippinarum (RpGST$\mu$): Cloning, mRNA expression, and conjugation assays*, 2014